# EUROPÄISCHE PATENTSCHRIFT

(11) **EP 1 498 083 B1**
(45) Veröffentlichungstag und Bekanntmachung des Hinweises auf die Patenterteilung: **29.10.2008**
(21) Anmeldenummer: 03715894.6
(22) Anmeldetag: 28.03.2003
(51) Int. Cl.: A61F 2/06

(54) **IMPLANTIERBARE HOHLE PROTHESE**
IMPLANTABLE HOLLOW PROSTHESIS
PROTHESE CREUSE IMPLANTABLE

(30) Priorität: 04.04.2002 RU 2002109439
(43) Veröffentlichungstag der Anmeldung: 19.01.2005
(73) Patentinhaber: Dyakov, Valery Evgenievich, St. Petersburg, 194356 (RU)
(72) Erfinder: DYAKOV, Valery Evgenievich, St.Petersburg, 194356 (RU); DUSHENKOV, Anatoly Stepanovich, St.Petersburg, 193232 (RU); KORTUNOV, Yury Anatolievich, St.Petersburg, 193171 (RU); FEDOTOVA, Lubov Mihailovna, St.Petersburg, 195176 (RU)
(74) Vertreter: Jeck, Anton
(86) Internationale Anmeldenummer: PCT/RU2003/000136
(87) Internationale Veröffentlichungsnummer: WO 2003/084438

(56) Entgegenhaltungen:
- EP-A1- 0 407 692
- WO-A-00/43052
- WO-A-95/05555
- WO-A-96/40001
- FR-A1- 2 699 066
- RU-C1- 2 117 459
- RU-C1- 2 128 024
- US-A- 5 413 597
- US-A- 5 607 478
- US-A- 5 843 171
- US-A- 5 910 168

## Beschreibung

Die Erfindung bezieht sich auf die medizinische Technik, und zwar auf Prothesen von Blutgefäßen und anderen Rohr- oder Hohlorganen, die in den menschlichen Organismus implantiert werden. Diese Prothesen können in der intravasalen Chirurgie, in der Chirurgie der Kanäle der extrasekretorischen Organe, in der chirurgischen Onkologie, Gastroenterologie, Urologie, Pneumologie und für die extraanatomische Verwendung eingesetzt werden.

In allen erwähnten Anwendungsbereichen gehört zu den wesentlichen Voraussetzungen für einen erfolgreichen Einsatz der Prothese deren Bioverträglichkeit, Haltbarkeit, Resistenz gegen eine Infektionsverbreitung, niedrige Thrombogenität und Blutdurchlässigkeit; diese Voraussetzungen finden in der Erhaltung der physisch-mechanischen Eigenschaften der Prothese bei der Integration der Prothese in den Organismus und im Fehlen einer Abstoßungsreaktion des Implantants seitens des Organismus ihren Ausdruck.

Eine der Forderungen, die an die für die Herstellung der Implantatprothesen vorgesehenen Materialien gestellt wird, ist die biologische und chemische Trägheit. Dieser Forderung werden hauptsächlich Polytetrafluorethylen (PTFE) oder die Kopolymere Tetrafluorethen (TFE) beispielsweise mit Hexafluoropropylen oder Perfluorvinylpropylenether gerecht.

### Stand der Technik

Durch die WO 00/43052 A1 ist eine Blutgefäßprothese aus ausgedehntem PTFE mit verbesserter Umströmung der Innenoberfläche bekannt. Diese Prothese besitzt eine glatte Innenoberfläche, welche die Okklusion der Blutkomponenten verhindert. Die Prothese ist als ein verlängertes Rohr aus extrudiertem und ausgedehntem PTFE mit durchschnittlicher Fibrillenlänge von 10-30 µm ausgeführt. Die Innenoberfläche der Prothese ist mit einer Folie aus ausgedehntem PTFE mit einer geringen durchschnitt- lichen Fibrillenlänge von 5µm und weniger bedeckt. Die Folienbeschichtung kann der ganzen Prothese entlang oder nur an den Stellen der vermutlichen Anastomose aus- gebildet werden. Die verbesserte Glätte der Prothese dient einer Verhinderung einer Thrombosenbildung.

WO 95/05555 A1 beschreibt eine Prothese, welche als dünnwandige Röhre mit einer Stärke von unter 0,25 mm aus porösem ausgedehntem PTFE ausgeführt ist, dessen Mikrostruktur eine Vielzahl von Fibrillen einschliesst, die vorwiegend parallel zu ein- ander ausgerichtet sind. Die Prothese umfasst mindestens zwei Schichten, die aus einer dünnen Folie von ausgedehntem PTFE ausgebildet sind. Die vorwiegende Fibrillenrichtung innerhalb der Innenschicht ist parallel der Röhrenlänge. In der Außenschicht sind sie die Fibrillen überwiegend normal zu der Röhrenlänge ausgerichtet. Die Röhre kann durch Aufwicklung von zwei Schichten aus der gleichen Folie hergestellt sein. Die Röhre kann an ihren Enden an den Stellen der vermutlichen Anastomose eine Manschette aus einem darauf aufgewickelten Band besitzen. Die Röhre schließt Verstärkungsschnüre bzw. Versteifungsrippen ein, die aus einem elastischen Material, hauptsächlich aus Metall ausgeführt sind und auf der Innen- oder Außenoberfläche oder zwischen den Schichten entlang der Röhre angeordnet sind. Als Verstärkungselement kann auch ein Metallstent eingesetzt werden. Die Röhre kann vorwiegend zur Beschichtung von Stenten, für Fibroskope oder zur intralaminaren (intravaskulären) Anordnung verwendet werden.

US 5,910,168 beschreibt eine Blutgefäßprothese aus einem biologisch verträglichen Material, vorzugsweise aus dem porösen ausgedehnten PTFE. Die Prothese umfasst eine 20 cm lange Hauptröhre mit einem Durchmesser von 6 mm, auf deren Außenoberfläche eine dicke, durch Aufwicklung von 5 bis 40 und mehr Schichten von poröser PTFE-Folie hergestellte Schicht ausgebildet ist. Diese Schicht ist derart ausgebildet, dass die einzelnen Teile der Oberfläche miteinander verbunden sind. Wesentliche Teile der Oberfläche können sich relativ zueinander bewegen. Die Schicht verhindert Blutungen, welche durch Durchstiche mittels chirurgischer Nadeln oder Dialysenadeln verursacht werden.

WO 96/40001 A1 beschreibt eine Blutgefäßprothese, welche von außen mit einem spiralartigen Folienband verstärkt ist. Die Prothese besitzt eine Innenröhre aus ausgedehntem PTFE und ist an ihrer Außenseite mit einem Folienband aus ausgedehntem PTFE umgewickelt. Die Prothese weist eine spiralförmige Versteifung aus nicht porösem PTFE auf. Der Bandaufwicklungswinkel und die Bandaufwicklungsganghöhe des Folienbandes und der Aufwicklungswinkel und der Ganghöhe der Versteifung fallen nicht.

Durch die EP 0293090 ist eine Blutgefäß-Hohlprothese aus PTFE bekannt, die in Form eines zylindrischen Rohrs ausgeführt ist und eine poröse Struktur in Form von Knoten hat, die mit Fibrillen verbunden sind. Die genannte Prothese ist durch Extrusion mit nachfolgender Extraktion hergestellt, die für die Gewinnung des porösen Erzeugnisses erforderlich ist.

Das in eine Richtung extrudierte PTFE hat jedoch eine ausgeprägte, faserige Struktur, wodurch beim Einsatz der Blutgefäßprothese geschwächte Stellen gebildet werden, die den Aneurysmen der natürlichen Blutgefäße ähnlich sind, und ein Einriss der Prothese und ein Bluterguss verursacht werden können. Außerdem kann bei der Anastomose das Ende der Prothese in der Längsrichtung bei Spannung des Verbindungsfadens zerreißen.

Eine der Lösungen zur Beseitigung des genannten Nachteils ist die Entwicklung und Nutzung von dünnwandigen (Dicke geringer als 0,25mm) Blutgefäßprothesen, die durch Aufwickeln einer feinen, gerichteten Folie aus PTFE auf einen Aufnahmedorn erzeugt werden. Die gerichtete Folie hat eine poröse Struktur in Form von Knoten, die mit Fibrillen verbunden sind, die der in der EP 0293090 beschriebenen Struktur ähnlich ist. Die Blutgefäßprothesen und die anderen rohrförmigen Implantate sind mindestens aus zwei Schichten der gerichteten PTFE-Folie hergestellt, die derart auf den Aufnahmedorn aufgewickelt sind, dass die Richtung der Fibrillen in den benachbarten Schichten

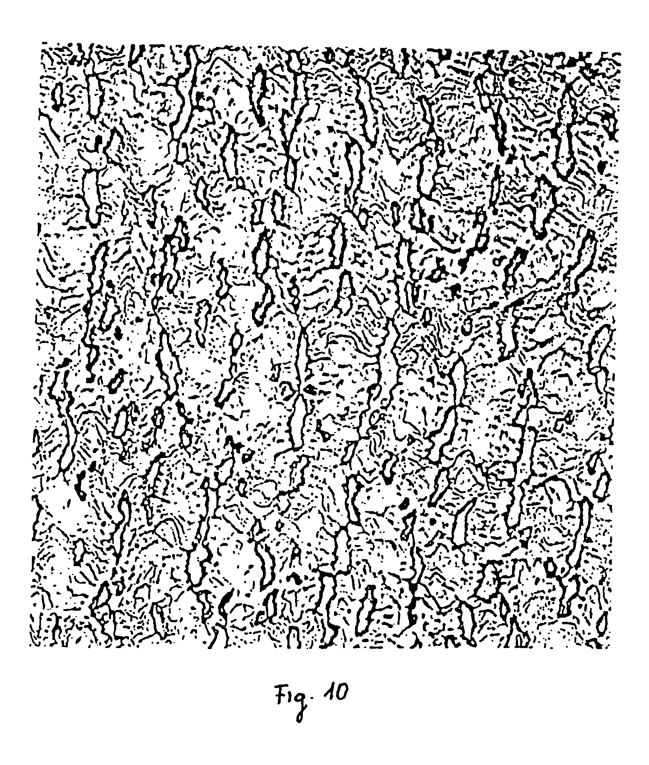

zueinander senkrecht stehen (USA-Patente 5972441, 6025044 und 602777a). Die Prothesen, die zwei oder mehrere Schichten der gerichteten PTFE-Folie enthalten, verfügen über eine hohe Verknüpfungshaltbarkeit und zerreißen beim Auflegen der Anastomosen nicht. Sie verfügen über Elastizität und können bis zu einem kleineren Durchmesser zusammengedrückt sein, so dass sie im zusammengedrückten Zustand über einen Katheter eingeführt werden können. Außerdem ermöglicht die Herstellung der Prothesen aus Folienschichten, die Prothesen mit Verzweigungen und Abzweigungen (Bifurkation) herzustellen. Sie bewahren aber ein Formgedächtnis, wie sich beim Biegen oder Verdrehen erkennen läßt. Auf dem implantierten Rohr wird ein Knick oder ein Verdrehen bei der Einwirkung einer geringsten, anregenden Kraft entstehen. Es ist nicht zufällig, dass die Prothesen, die als die besten Beispiele der Verwirklichung in den erwähnten Patenten vorgestellt sind, innere Verstärkungselemente (Fig. 10 in allen oben erwähnten Patenten) enthalten, die das Biegen oder Verdrehen unter mechanischer Einwirkung verhindern. Außerdem ist die Kontrolle des Spannungsgrads während der Operationsdurchführung bei Verwendung von dünnwandigen Prothesen kompliziert.

Es ist eine Implantathohlprothese aus dem (Ko)Polymer von Tetrafluorethen bekannt, deren Struktur in Form von Knotenelementen, die mit Fibrillen verbunden sind, und in Form von Elementen der Hohlräume mit der Vereinigung der Elemente in einem dreidimensionalen Netz ausgebildet ist. Die Implantathohlprothese enthält einen Körper, der mindestens aus einer Schicht geformt ist, die durch Aufwickeln eines Bands auf einen Dorn gebildet ist, das aus einem Material, das einen Volumenanteil der Hohlräume von 25-94%, eine spezifische Oberfläche der Hohlräume von 0,1-9,0 µm²/µm³, einen durchschnittlichen Abstand zwischen den Hohlräumen in der Größe von 1,5-50µm und eine mittlere Volumensehne von 0,4-30µm aufweist, oder aus einem Material hergestellt ist, das einen Volumenanteil der Hohlräume von 1-35%, eine spezifische Oberfläche der Hohlräume von 0,5-20µm²/µm³, einen durchschnittlichen Abstand zwischen den Hohlräumen in der Größe von 0,5-15µm und eine mittlere Volumensehne von 0,1-10µm aufweist (RF-Patent 2128024).

Die erwähnte Prothese hat eine Wanddicke von 0,01-0,25mm, kann mit Verzweigungen und Abzweigungen (Bifurkation) ausgeführt sein oder mehrere (zwei und mehr) Schichten der aufgewickelten Folie enthalten; dabei kann die Richtung der Fibrillen in den benachbarten Schichten zueinander senkrecht stehen.

Die Prothese gewährleistet eine hohe Durchlässigkeit für die Gewebe des Organismus im Laufe ihres Anheilens im Organismus, wodurch die Funktion des zu ersetzenden Organs (Blutgefäß) erfüllt und eine ausreichend hohe Verknüpfungshaltbarkeit gewährleistet wird.

Aber die elastischen Eigenschaften der Wand einer solchen Prothese sind von den Eigenschaften der Wand der nativen Arterie sehr weit entfernt. Infolgedessen leitet die Prothese die Pulswelle schlecht durch. Die Störung der Pulswelle trägt zur Entwicklung einer Hyperplasie der Gefäßintima im Bereich der Anastomose bei, wodurch dann eine Prothesenthrombose entstehen kann.

### Kurzfassung der Erfindung

Die Erfindung hat sich zur Aufgabe gestellt, die elastischen Eigenschaften der Prothese zu erhöhen, so dass die Eigenschaften der Prothese den Eigenschaften der nativen Arterie näher gebracht werden.

Gemäß der Erfindung wird eine Implantathohlprothese aus Polymermaterial vorgeschlagen, dessen Struktur in Form von Knotenelementen, die mit Fibrillen verbunden sind, und in Form von Elementen der Hohlräume mit der Vereinigung der Elemente in einem dreidimensionalen Netz gebildet ist. Die lmplantathohlprothese enthält einen Körper, der mindestens aus einer Schicht geformt ist, die durch Aufwickeln eines Bands gebildet ist, das aus einem Material hergestellt ist, das aus der Gruppe ausgewählt wird, die ein Material, das einen Volumenanteil der Hohlräume von 25-94%, eine spezifische Oberfläche der Hohlräume von 0,1-9,0 µm²/µm³, einen durchschnittlichen Abstand zwischen den Hohlräumen in der Größe von 1,5-50µm und eine mittlere Volumensehne von 0,4-30µm aufweist, oder ein Material enthält, das einen Volumenanteil der Hohlräume von 1-35%, eine spezifische Oberfläche der Hohlräume von 0,5-20µm²/µm3, einen durchschnittlichen Abstand zwischen den Hohlräumen in der Größe von 0,5-15µm, eine mittlere Volumensehne von 0,1-10µm aufweist und das zusätzlich eine innere, poröse Karkasse mit einer Wanddicke von 0,2-2,0mm enthält, die aus einem porösen Polymermaterial hergestellt ist, das einen Volumenanteil von 30-90%, eine spezifische Oberfläche der Hohlräume von 0,1-0,9µm²/µm³, einen durchschnittlichen Abstand zwischen den Hohlräumen in der Größe von 0,5-50µm und eine mittlere Volumensehne von 1-30µm aufweist.

Je nach der Bestimmung und dem Durchmesser der Implantathohlprothese kann die innere, poröse Karkasse eine Wanddicke von 0,2 bis 2,0mm aufweisen. So beträgt in den Blutgefäßprothesen mit einem Durchmesser von 2-8mm die Dicke der inneren, porösen Karkasse insgesamt 0,20-0,75mm; in Prothesen mit einem größeren Durchmesser (10-30mm) beträgt die Dicke der inneren, porösen Karkasse 0,75-2,00mm.

Die innere, poröse Karkasse ist durch Extrusion von PTFE oder Kopolymeren Tetrafluorethen mit Hexafluoropropylen oder Perfluorvinylpropylenether mit einem nachfolgenden Auszug um 300-1000% geformt. Der Auszug bildet die Struktur, die aus den Knotenelementen, die mit den Fibrillen verbunden sind, und aus den Elementen der Hohlräume besteht, die in das dreidimensionale Netz eingebunden sind. Diese Struktur hat einen Volumenanteil der Hohlräume von 30-90%, eine spezifische Oberfläche der Hohlräume von 0,1-0,9µm²/µm³, einen durchschnittlichen Abstand zwischen den Hohlräumen in der Größe von 0, 5-50µm und eine mittlere Volumensehne von 1-30µm.

Die Hülle der Implantathohlprothese, die durch das Aufwickeln des Bands gebildet ist, das die oben genannte Struktur aufweist, enthält 1-6 Schichten des Bands, besser 2-6 Schichten und vorzugsweise 4-6 Schichten. Der Anlaufwinkel des Bands in jeder Schicht (im Verhältnis zu der Längsachse der Prothese) ist größer als 0° und geringer als oder gleich 90°. Das Aufwickeln des Bands in den benachbarten Schichten kann unter dem gleichen oder unter einem unterschiedlichen Winkel oder kreuzförmig ausgeführt werden. Der Aufwickelschritt des Bands ist gleich der oder geringer als die Breite des Bands.

Das Band, das die Hülle der Implantathohlprothese bildet, wird so hergestellt, wie es im RF-Patent 2128024 beschrieben ist. Es wurde festgestellt, dass die besten Ergebnisse das Aufwickeln von doppelaxial gerichteten Bändern liefern, die die folgende Struktur aufweisen: einen Volumenanteil der Hohlräume von 45-94%, eine spezifische Oberfläche der Hohlräume von 0,1-0,6µm²/µm³, einen durchschnittlichen Abstand zwischen den Hohlräumen von 5-45µm und eine mittlere Volumensehne von 5-30µm. Die Schichten können durch das Aufwickeln des Bands von jeder oben genannten Struktur geformt werden. Die Hülle der Implantathohlprothese kann Schichten mit verschiedenen Strukturen enthalten, die oben genannt sind.

Die lmplantathohlprothese kann auf der Außenseite ein Verstärkungselement aufweisen, das in Form von einzelnen Ringen oder Spiralen ausgeführt ist. Die Verstärkungsringe oder -spiralen sind an der Außenhülle der Prothese angebracht. Die Prothese kann über eine zusätzliche Außenhülle mit dem Verstärkungeelement(en) verfügen, das (die) an der Hülle befestigt ist (sind). Die Hülle ist an der Prothese mit der Möglichkeit der Verschiebung (Gleiten) längs der Längsachse der Prothese angeordnet. Die Prothese, die das Verstärkungselement enthält, kann eine zusätzliche Hülle besitzen, die durch Aufwickeln eines Bands gebildet ist und von oben das Verstärkungselement abdeckt.

Das Polymermaterial, aus dem die Implantathohlprothese hergestellt ist, ist Polytetrafluorethylen oder ein Kopolymer Tetrafluorethen mit Hexafluoropropylen oder Perfluorvinylpropyleriether, das nicht mehr als 2% Komonomer, vorzugsweise Polytetrafluorethylen, enthält.

Die Verstärkungselemente in Form von Ringen oder Spiralen können aus Polymer (PTFE) oder Kopolymeren TFE mit Hexafluoropropylen oder Perfluorvinylpropylenether) oder aus Metall in einer Polymerhülle oder aus Kohlefaser, vorzugsweise Polymerfaser, bestehen.

### Ausführungsbeispiele der Erfindung

Die Erfindung wird nun anhand von Ausführungsbeispielen näher erläutert. Prothesen gemäß der Erfindung sind in den folgenden Figuren 1-11 dargestellt. Es zeigen im Einzelnen:
- Fig. 1: eine Hohlprothese, deren Körper aus einer Schicht eines Bands hergestellt ist, das auf einer porösen Karkasse mit einem Schritt aufgewickelt ist, der der Bandbreite gleich ist,
- Fig. 2: eine Hohlprothese, deren Körper aus einer Schicht eines Bands hergestellt ist, das auf eine poröse Karkasse mit einem Schritt aufgewickelt ist, der kleiner als die Bandbreite ist,
- Fig. 3: eine Hohlprothese, deren Körper aus einigen (vier) Schichten eines Bands hergestellt ist, das auf eine poröse Karkasse mit einer kreuzförmigen Richtung des Aufwickelns in den benachbarten Schichten aufgewickelt ist,
- Fig. 4: eine Hohlprothese, die mit einer äußeren Spirale verstärkt ist,
- Fig. 5: eine Hohlprothese mit einer zusätzlichen "gleitenden" Hülle, die mit einer Verstärkungsspirale versehen ist,
- Fig. 6: eine Hohlprothese mit einer zusätzlichen "gleitenden" Hülle und einem Verstärkungselement, das von oben mit der Hülle abgedeckt ist,
- Fig. 7: eine Hohlprothese, die mit Außenringen verstärkt ist,
- Fig. 8: eine Hohlprothese, deren Körper aus sechs Schichten einer Folie hergestellt ist, die auf eine poröse Karkasse aufgewickelt ist, wobei die Richtung des Aufwickelns in den benachbarten Schichten kreuzförmig ist,
- Fig. 9: einen unter einem Mikroskop entstandenen Längsschnitt der Prothese gemäß der Erfindung,
- Fig. 10: ein Bild der inneren Oberfläche der Prothese und
- Fig. 11: ein Bild einer doppelaxial gerichteten Folie, aus der die Außenhülle der Prothese hergestellt ist.

Die Prothese schließt eine poröse Karkasse 1 ein, die aus PTFE oder dem Kopolymer TFE ausgeführt ist, deren Struktur gekennzeichnet ist durch:
- zwei verknüpfte, gegenseitig durchdringende Matrizen,
- eine Polymermatrix in Form von Knoten, die mit Fibrillen verbunden sind,
- und eine Matrix des Hohlraums, dessen Elemente in ein dreidimensionales Netz eingebunden sind.

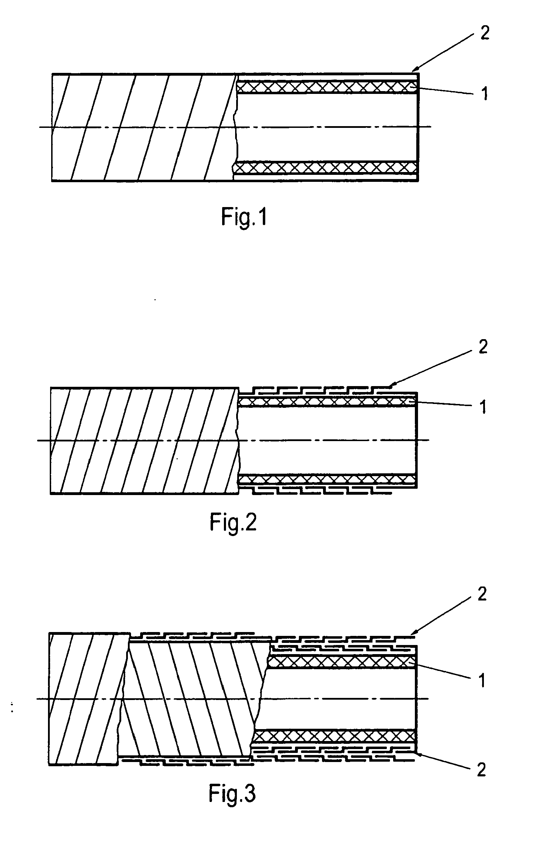

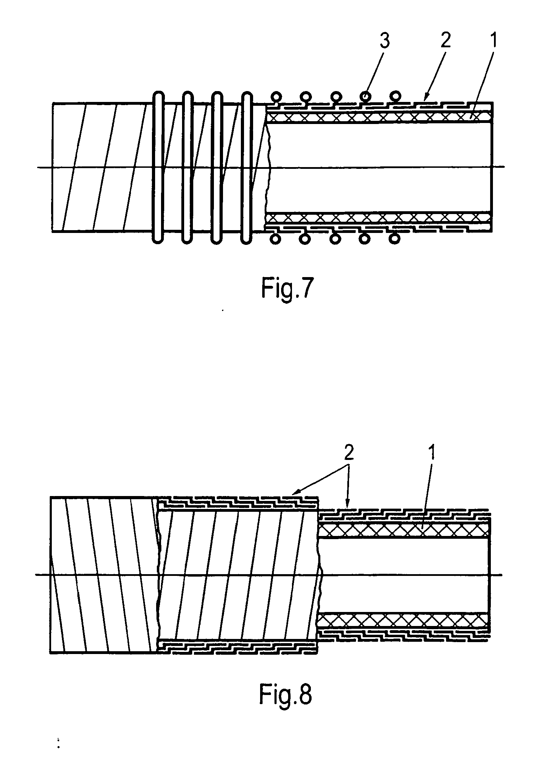

Auf die poröse Karkasse 1 ist die Folienschicht mit einer Dicke von nicht weniger als 0,005mm aufgewickelt; die Folie ist in Streifen zerschnitten. Die Folie ist auch aus Polytetrafluorethylen oder einem Kopolymer Tetrafluorethen mit Hexafluoropropylen oder Perfluorvinylpropylenether hergestellt, das bis 2% Komonomer enthält. Die Struktur der Folie zeichnet sich auch durch zwei gegenseitig durchdringende Matrizen, eine Polymermatrix in Form von Knoten, die mit den Fibrillen verbunden sind, und durch eine Matrix des Hohlraums, dessen Elemente in das dreidimensionale Netz eingebunden sind. Die Folie ist unter einem Winkel von 0° bis 90° aufgewickelt; der Aufwickelschritt ist geringer (Fig. 2) als die Bandbreite oder gleich der Bandbreite (Fig. 1). Das Band kann einschichtig, wie es in den Figuren 1 und 2 gezeigt ist, oder in mehren Schichten gewickelt werden, wie es in den Figuren 3 und 8 gezeigt ist. Im letzten Fall stimmen die Aufwickelwinkel in den benachbarten Schichten nicht überein; die Schichten sind kreuzförmig aufgewickelt.

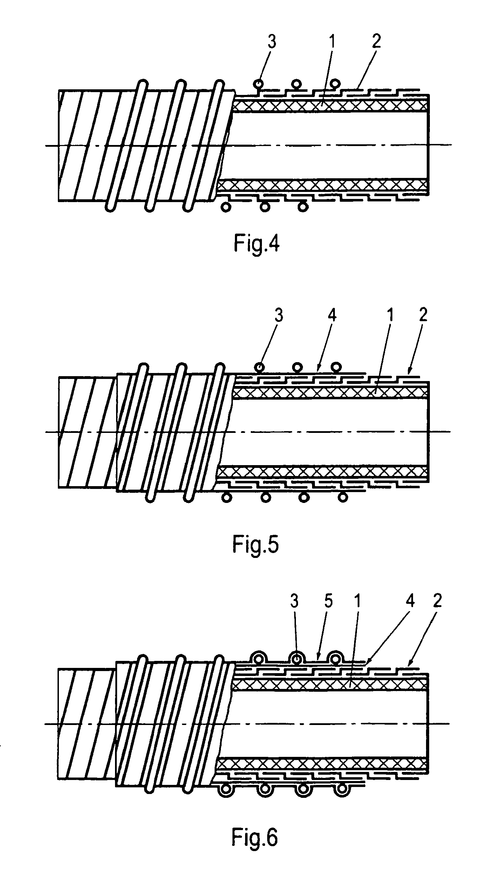

Auf der Außenseite kann die Prothese ein Verstärkungselement (Verfestigungselement) aufweisen, das in Form einer Spirale 3 oder von Ringen 3 (s. Figuren 4 und 7) ausgeführt ist, das auf einer Schicht 2 untergebracht ist, die durch das Aufwickeln des Bands gebildet ist, und das an dieser Schicht befestigt ist. Das Verstärkungselement kann aus PTFE oder einem Kopolymer TFE mit Hexafluoropropylen hergestellt werden. Das Verstärkungselement 3 kann an einer zusätzlichen Hülle 4 (s. Fig. 5) befestigt werden, die aus dem gleichen doppelaxial gerichteten PTFE-Band ausgeführt und auf der Prothese oberhalb der Hülle mit der Möglichkeit der Bewegung längs der Prothesenachse ("gleitende" Hülle) untergebracht ist. Das Verstärkungselement 3 kann von oben mit einer zusätzlichen Hülle 5 (Fig. 6) zugedeckt werden, die aus dem gleichen doppelaxial gerichteten PTFE-Band ausgeführt werden kann. Im letzten Fall kann das Verstärkungselement aus Metall, z. B. Stahl, Titan, der Legierung Nitinol usw., in Form einer Spirale oder eines Netzes ausgeführt werden.

Die Raumparameter der Karkasse und der Hüllen der Prothese gemäß der Erfindung sind nach einer bekannten Methodik festgestellt (Panteleev W.G., Ramm K.S., "Neoorganische Materialien", 1986, Band 22, Nr. 12, S. 1941-1951; Awtangilov G.G. und andere, "Die Systemstereometrie in der Forschung des pathologischen Prozesses", M., Medizin, 1981; Tschernjawsky K.S. "Sterelogie in der Metallkunde", M., Metallurgie , 1977).

Gemäß der Definition hat das Material der Karkasse einen Volumenanteil der Hohlräume von 30-90%, eine spezifische Oberfläche der Hohlräume von 0,1-0,9µm²/µm³, einen durchschnittlichen Abstand zwischen den Hohlräumen in der Größe von 0,5-50µm und eine mittlere Volumensehne von 1-30µm.

Die Materialstruktur der Hülle, die aus der doppelaxial gerichteten Folie hergestellt ist, hat: einen Volumenanteil der Hohlräume von 25-94%, eine spezifische Oberfläche der Hohl räume von 0,1-9,0µm²/µm³, einen durchschnittlichen Abstand zwischen den Hohlräumen von 1,5-50µm und eine mittlere Volumensehne von 0,4-30µm bzw. einen Volumenanteil der Hohlräume von 45-94%, eine spezifische Oberfläche der Hohlräume von 0,1-0,6µm²/µm³, einen durchschnittlichen Abstand zwischen den Hohlräumen in der Größe von 5-45µm und eine mittlere Volumensehne in der Größe von 5-30µm bzw. einen Volumenanteil der Hohlräume von 1-35%, eine spezifische Oberfläche der Hohlräume von 0,5-20µm²/µm³, einen durchschnittlichen Abstand zwischen den Hohlräumen in der Größe von 0,5 -15µm und eine mittlere Volumensehne von 0,1-10µm.

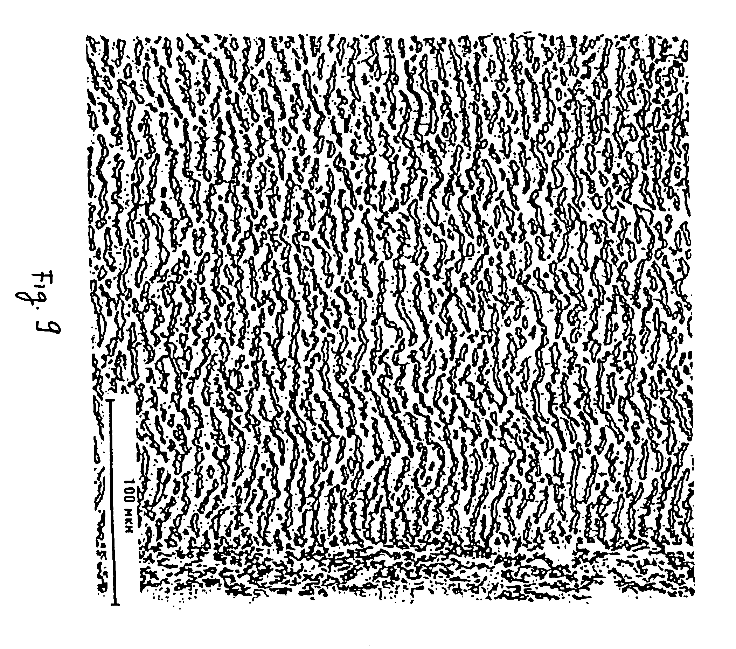

In Fig. 9 ist ein Längsschnitt durch die Prothese wie in Fig. 3 dargestellt, mit einer mehrschichtigen Hülle, die kreuzförmig aufgewickelt ist. Die Dicke der Karkasse beträgt in diesem Fall 0,5mm (500µm), während die Dicke der Hülle 55µm beträgt. Deutlich sind das dreidimensionale Netz, das durch die Hohlräume gebildet ist, und die Knoten der Polymermatrix zu erkennen.

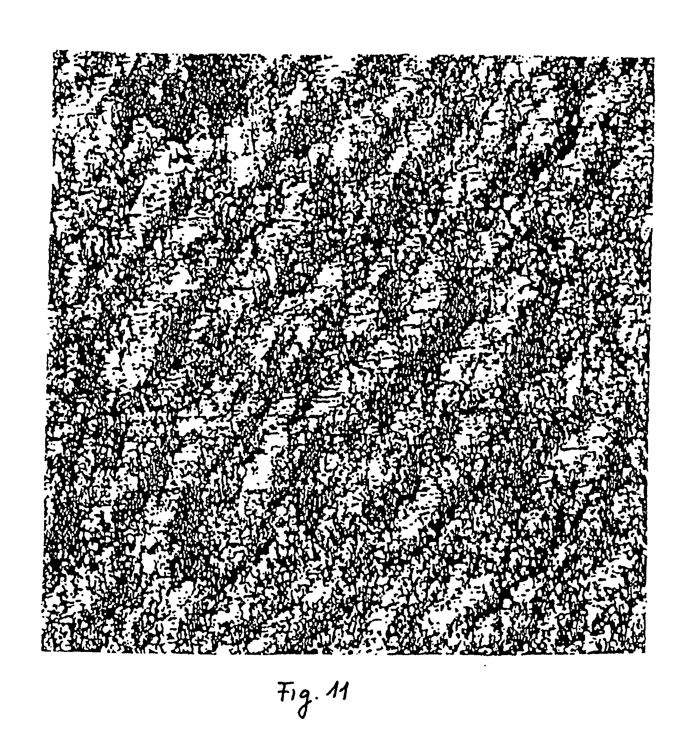

Die Bilder der inneren Oberfläche der Prothese (Fig. 10) und der Folie, die die Außenschicht der Prothese (Fig. 11) bilden, zeigen ebenfalls das Vorhandensein der genannten Struktur.

Die dynamischen, elastischen Eigenschaften (Geschwindigkeit der Pulswelle) von Mustern der Prothese gemäß der Erfindung im Vergleich zu den Mustern der nativen Blutgefäße wurden nach der Methodik untersucht, die im Labor der medizinischen Polymere vom Forschungszentrum der kardiovaskulären Chirurgie von A.N. Bakulev der Akademie der medizinischen Wissenschaften Russlands (Moskau) entwickelt wurde. Die Testergebnisse sind in der folgenden Tabelle dargestellt.

**Tabelle**

| Aufbau und Struktur der Muster der Implantatprothese und die Geschwindigkeit des Durchgangs der Pulswelle | | | | | | | | | | | |
|---|---|---|---|---|---|---|---|---|---|---|---|
| Durchme sser der Prothese, mm | Kenndaten des porösen Karkasse | | | | | Kenndaten der porösen Hülle | | | | | Geschwindigkeit der Pulswelle s, m/s |
| | d 1) mm | d 1) % | d 3) µm ² /µm ³ | d 1) µm | d 1) µm | Anzahl der Schicht en | 2) % | 3) µm ² /µm ³ | 4) µm | 5) µm | |
| 6 | 0,45 | 78 | 0,21 | 15 | 30 | 1 | 80 | 0,29 | *5,7* | 29,5 | 7,9 |
| 6 | 0,33 | 57 | 0,32 | 16 | 12 | 1 | 33 | 12 | 14 | 3,5 | 12,3 |
| 6 | 0,45 | 75 | 0,20 | 14 | 27 | 4 | 80 | 0,29 | 5,7 | 29,5 | 9,6 |
| 12 | 0,85 | 79 | 0,25 | 10 | 29 | 4 | 80 | 0,29 | 5,7 | 29,5 | 12,4 |
| 2 | 0,23 | 65 | 0,28 | 12 | 18 | 2 | 86 | 0,29 | 4,2 | 28,7 | 5,3 |
| 32 | 1,95 | 68 | 0,3 | 12,8 | 16,7 | 6 | 82 | 0,26 | 4,9 | 27,9 | 17,3 |
| 6 | Ohne Karkasse | | | | | 4 | 28 | 11,3 | 10,8 | 2,0 | 23,5 |

| | | | | | | | | | | | |
|---|---|---|---|---|---|---|---|---|---|---|---|
| 1) Wanddicke der Karkasse 2) Volumenanteil der Hohlräume 3) spezifische Oberfläche der Hohlräume 4) durchschnittlicher Abstand zwischen den Hohlräumen 5) mittlere Volumensehne. | | | | | | | | | | | |

Die Fortpflanzungsgeschwindigkeit der Pulswelle im nativen Blutgefäß (Schenkelarterie in vitro) mit einem Durchmesser von 6mm beträgt 8,0±0,5m/s. Zum Vergleich sind die Daten für die Prothese angeführt, die keine Karkasse enthalten (nach dem Prototyp).

Wie es Sich aus den Daten der Tabelle erkennen läßt, nähert sich die Geschwindigkeit der Pulswelle in der angemeldeten Implantatprothese der Geschwindigkeit der Pulswelle in den nativen Blutgefässen und ist niedriger als in der Prothese gemäß dem Prototyp. Die Geschwindigkeit der Pulswelle im Gefäß mit einem Durchmesser von 32mm (Prothese der Aorta) ist höher; dies entspricht der Geschwindigkeit der Pulswelle in ähnlichen Gefäßen.

Die Bauchaorten von 25 Nichtrassehunden wurden mit der Implantathohlprothese gemäß der Erfindung prothetisiert, wobei die Prothese einen Durchmesser von 6mm aufwies. Die Dauer der Beobachtung betrug einen Monat bis dreizehn Monate. Die Prothese ließ sich leicht in Übereinstimmung mit den Besonderheiten der Anastomose modellieren, leicht mit der Nadel durchstechen und gut an den Rand des Gefäßes anpassen, wobei die Dichtigkeit durch Einziehen eines Fadens gewährleistet wurde. Die Wand der Prothese ließ sich nach dem Anfertigen der Naht nicht durchstechen und entflechten.

Bei Beginn der Blutströmung war nur in einigen Fällen eine geringe Blutung zu beobachten, die schnell mit einem Tupfer zu stillen war. Weder in der Nachoperationszeit noch in der Folgezeit der Beobachtung war bei den operierten Hunden eine Thrombose zu verzeichnen. Durch Tastuntersuchung ließ sich deutlich das Pulsieren der Schenkelarterie feststellen.

Bei der Entnahme des Materials hatten alle Prothesen eine Form und Masse, die den Ausgangsdaten entsprachen. In keinem Fall konnte ein Hereinziehen der Prothese in den Narbenperiprozess festgestellt werden. Nach 14 Monaten ließ sich das Transplantat, das mit einer dünnen, festen Neuadventitia bedeckt war, gut durchzeichnen und war nicht mit den umliegenden Organen zusammengewachsen.

Nach 5-7 Monaten deckte die Neuintima der Transplantate komplett die innere Oberfläche der Prothese ab; der Übergang der Intima von der Aorta in die Neuintima der Prothese war glatt. Anzeichen von Hyperplasie der Gefäßintima im Bereich der Anastomose fehlten.

### Industrielle Anwendbarkeit

Die Implantathohlprothese gemäß der Erfindung kann in verschiedenen Bereichen der Chirurgie zum Einsatz kommen, beispielsweise in der intravasalen Chirurgie, in der Pneumologie, Urologie, Gastroenterologie usw. Die Implantathohlprothese kann von der medizinischen Industrie hergestellt werden.

## Patentansprüche

1. Implantathohlprothese aus Kunststoff, deren Struktur in Form von Knotenelementen, die mit Fibrillen verbunden sind, sowie in Form von Elementen der Hohlräume mit der Vereinigung der Elemente in einem dreidimensionalen Netz ausgebildet ist, und mit einem Prothesenkörper [2], der mindestens aus einer Schicht gebildet ist, die durch Aufwickeln eines Bands gebildet und aus einem Material hergestellt ist, das aus der Gruppe ausgewählt ist, die ein Material, das einen Volumenanteil der Hohlräume von 25-94%, eine spezifische Oberfläche der Hohlräume von 0,1 - 9,0 µm²/µm³, einen durchschnittlichen Abstand zwischen den Hohlräumen in der Größe von 1,5 - 50 µm und eine mittlere Volumensehne von 0,4 - 30 µm aufweist, und ein Material einschließt, das einen Volumenanteil der Hohlräume von 1 - 35%, eine spezifische Oberfläche der Hohlräume von 0,5 - 20 µm²/µm³, einen durchschnittlichen Abstand zwischen den Hohlräumen in der Größe von 0,5 - 15 µm und eine mittlere Volumensehne von 0,1 - 10 µm aufweist,
**dadurch gekennzeichnet,**
**dass** die Implantathohlprothese zusätzlich eine innere, poröse Karkasse [1] mit einer Wanddicke von 0,2 - 2,0 mm enthält, die aus einem Polymermaterial hergestellt ist, dessen Struktur in Form von Knotenelementen, die mit Fibrillen verbunden sind, und in Form von Elementen der Hohlräume mit der Vereinigung
der Elemente in einem dreidimensionalen Netz ausgeführt ist, das einen Volumenanteil der Hohlräume von 30 - 90%, eine spezifische Oberfläche der Hohlräume von 0,1 - 0,9 µm²/µm³, einen durchschnittlichen Abstand zwischen den Hohlräumen in der Grösse von 0,5 - 50 µm und eine mittlere Volumensehne von 1 - 30 µm aufweist.

2. Implantathohlprothese nach Anspruch 1,
**dadurch gekennzeichnet,**
**dass** sie durch Aufwickeln von zwei bis sechs Schichten eines Bands aus Polymermaterial gebildet ist.

3. Implantathohlprothese nach Anspruch 1,
**dadurch gekennzeichnet,**
**dass** sie durch Aufwickeln von vier bis sechs Schichten eines Bands aus Polymermaterial gebildet ist.

4. Implantathohlprothese nach einem der Ansprüche 1 bis 3,
**dadurch gekennzeichnet,**
**dass** der Bandaufwickelwinkel mehr als 0 DEG und geringer als oder gleich 90 DEG ist.

5. Implantathohlprothese nach Anspruch 2 oder 3,
**dadurch gekennzeichnet,**
**dass** das Aufwickeln des Bands in den benachbarten Schichten kreuzförmig erfolgt.

6. Implantathohlprothese nach einem der Ansprüche 1 bis 3,
**dadurch gekennzeichnet,**
**dass** der Aufwickelschritt gleich der oder geringer als die Bandbreite ist.

7. Implantathohlprothese nach Anspruch 1,
**dadurch gekennzeichnet,**
**dass** der Körper der Prothese durch das Aufwickeln eines doppelaxial gerichteten Bands gebildet ist, das aus einem Material hergestellt ist, das einen Volumenanteil der Hohlräume von 45 - 94%, eine spezifische Oberfläche der Hohlräume von 0,1 - 0,6 µm²/µm³, einen durchschnittlichen Abstand zwischen den Hohlräumen in der Größe von 5 - 45 µm und eine mittlere Volumensehne von 5 -30 µm aufweist.

8. Implantathohlprothese nach Anspruch 1,
**dadurch gekennzeichnet,**
**dass** sie ein Verstärkungselement (3) einschließt, das in Form von Ringen ausgebildet ist, die auf der Außenoberfläche der Prothese befestigt sind.

9. Implantathohlprothese nach Anspruch 1,
**dadurch gekennzeichnet,**
**dass** sie ein Verstärkungselement (3) einschließt, das in Form von Spiralen ausgebildet ist, die auf der Außenoberfläche der Prothese befestigt sind.

10. Implantathohlprothese nach Anspruch 8 oder 9,
**dadurch gekennzeichnet,**
**dass** die Verstärkungselemente (3) an der zusätzlichen Außenhülle (4) befestigt sind, die durch Aufwickeln des Bands aus Polymermaterial gebildet und längs der Prothesenlängsachse bewegbar angebracht ist.

11. Implantathohlprothese nach Anspruch 8 oder 9,
**dadurch gekennzeichnet,**
**dass** sie zusätzlich eine Hülle (5) aufweist, die durch Aufwickeln eines Bands aus Polymermaterial gebildet ist, das oberhalb des Verstärkungselements (3) befestigt ist.

12. Implantathohlprothese nach Anspruch 1,
**dadurch gekennzeichnet,**
**dass** das Band aus einem Polymermaterial hergestellt ist, das aus der Gruppe ausgewählt ist, die Polytetrafluorethylen und das Kopolymer Tetrafluorethen mit Hexafluoropropylen enthält, das einen Gehalt von nicht mehr als 2 % der Massen des Kopolymers Tetrafluorethen mit Perfluorvinylpropylenether aufweist.

13. Implantathohlprothese nach Anspruch 1,
**dadurch gekennzeichnet,**
**dass** die innere Karkasse aus Polymermaterial hergestellt ist, das aus der Gruppe ausgewählt ist, die Polytetrafluorethylen und die Kopolymere Tetrafluorethen mit Hexafluoropropylen sowie Tetrafluorethen mit Perfluorvinylpropylenether mit einem Gehalt des Komonomers von nicht mehr als 2% der Massen aufweist.

## Claims

1. Implantable hollow prosthesis made of plastics material, the structure of which is realised in the form of node element that are connected to fibrils and in the form of hollow space elements that, when joined, form a three-dimensional network, and having a prosthesis body (2) that is formed from at least one layer, which is formed by winding a tape that is produced from one material that is selected from the group that includes a material with a hollow space volume fraction of between 25 and 94%, a specific hollow space surface of between 0.1 and 9.0 µm²/µm³, an average spacing between the hollow spaces in the order of between 1.5 and 50 µm and a mean volume chord of between 0.4 and 30 µm, and includes a material with a volume fraction of between 1 and 35%, a specific hollow space surface of between 0.5 and 20 µm²/µm³, an average spacing between the hollow spaces in the order of between 0.5 and 15 µm and a mean volume chord of between 0.1 and 10µm, **characterised in that** the implantable hollow prosthesis also includes an inner porous casing (1) with a wall thickness of between 0.2 and 2.0 mm, the said casing being produced from a polymer material, the structure of which is realised in the form of node elements that are connected to fibrils and in the form of hollow space elements that, when joined, form a three-dimensional network, which includes a hollow space volume fraction of between 30 and 90%, a specific hollow space surface of between 0.1 and 0.9 µm²/µm³, an average spacing between the hollow spaces in the order of between 0.5 and 50 µm and a mean volume chord of between 1 and 30 µm.

2. Implantable hollow prosthesis according to claim 1, **characterised in that** the said implantable hollow prosthesis is formed by winding two to six layers of a tape made of polymer material.

3. Implantable hollow prosthesis according to claim 1, **characterised in that** the said implantable hollow prosthesis is formed by winding four to six layers of a tape made of polymer material.

4. Implantable hollow prosthesis according to one of claims 1 to 3, **characterised in that** the tape winding angle is in excess of 0° and is less than or equal to 90°.

5. Implantable hollow prosthesis according to claim 2 or 3, **characterised in that** the winding of the tape is effected in a crosswise manner in the adjacent layers.

6. Implantable hollow prosthesis according to one of claims 1 to 3, **characterised in that** the winding step is equal to or less than the tape width.

7. Implantable hollow prosthesis according to claim 1, **characterised in that** the body of the prosthesis is formed by the winding of a tape, which is directed in a double axial manner and is produced from a material that includes a hollow space volume fraction of between 45 and 94%, a specific hollow space surface of between 0.1 and 0.6 µm²/µm³, an average spacing between the hollow spaces in the order of between 5 and 45 µm and a mean volume chord of between 5 and 30 µm.

8. Implantable hollow prosthesis according to claim 1, **characterised in that** the said implantable hollow prosthesis includes a reinforcing element (3), which is in the form of rings that are secured on the outside surface of the prosthesis.

9. Implantable hollow prosthesis according to claim 1, **characterised in that** the said implantable hollow prosthesis includes a reinforcing element (3), which is in the form of spirals that are secured on the outside surface of the prosthesis.

10. Implantable hollow prosthesis according to claim 8 or 9, **characterised in that** the reinforcing elements (3) are secured to the additional outside sleeve (4), which is formed by winding the tape made from polymer material and is attached so as to be displaceable along the longitudinal axis of the prosthesis.

11. Implantable hollow prosthesis according to claim 8 or 9, **characterised in that** the said implantable hollow prosthesis additionally includes a sleeve (5), which is formed by winding a tape made of polymer material that is secured above the reinforcing element (3).

12. Implantable hollow prosthesis according to claim 1, **characterised in that** the tape is produced from a polymer material that is selected from the group that includes polytetrafluoroethylene and the copolymer tetrafluorethane with hexafluoro-propylene, which has a content of tetrafluoroethylene with perfluorvinylpropylene ether that is no more than 2% of the copolymer mass.

13. Implantable hollow prosthesis according to claim 1, **characterised in that** the inner casing is produced from polymer material, which is selected from the group that includes polytetrafluoroethylene and the copolymer tetrafluorethane with hexafluoro-propylene as well as tetrafluoroethylene with perfluorvinylpropylene ether with a content of the comonomer of more than 2% of the mass.

## Revendications

1. Prothèse creuse implantable, en matière plastique, dont la structure est sous forme d'éléments de noeuds qui sont reliés par des fibrilles, ainsi que sous forme d'éléments des pores avec la réunion des éléments dans un réseau en trois dimensions, et comprenant une pièce ( 2 ) de prothèse, qui est formée d'au moins une couche formée en enroulant une bande et en un matériau qui est choisi dans le groupe qui comprend un matériau qui a une proportion en volume des pores de 25 à 94 %, une surface spécifique des pores de 0,1 à 9,0 µm²/µm³, une distance moyenne entre les pores de l'ordre de 1,5 à 50 µm²/µm³ et une valeur moyenne de la corde du volume de 0,1 à 10 µm,
**caractérisée en ce que** la prothèse implantable comporte en outre une carcasse ( 1 ) intérieure, poreuse, ayant une épaisseur de paroi de 0,2 à 2 mm, qui est en une matière polymère dont la structure est réalisée sous la forme d'éléments de noeuds qui sont reliés par des fibrilles et sous la forme d'éléments des pores avec la réunion des éléments dans un réseau tridimensionnel, qui a une proportion en volume des pores de 30 à 90 %, une surface spécifique des pores de 0,1 à 0,9 µm²/µm³, une distance moyenne entre les pores de l'ordre de 0,5 à 50 µm et une valeur moyenne de la corde du volume de 1 à 30 µm.

2. Prothèse creuse implantable selon la revendication 1,
**caractérisée**
**en ce qu'**elle est formée par enroulement de 2 à 6 couches d'une bande en matière polymère.

3. Prothèse creuse implantable selon la revendication 1,
**caractérisée**
**en ce qu'**elle est formée par enroulement de 4 à 6 couches d'une bande en matière polymère.

4. Prothèse creuse implantable selon l'une des revendications 1 à 3,
**caractérisée**
**en ce que** l'angle d'enroulement de la bande est supérieur à 0 DEG et inférieur ou égal à 90 DEG.

5. Prothèse creuse implantable selon la revendication 2 ou 3,
**caractérisée**
**en ce que** l'enroulement de la bande dans les couches voisines s'effectue en croix.

6. Prothèse creuse implantable selon l'une des revendications 1 à 3,
**caractérisée**
**en ce que** le pas d'enroulement est inférieur ou égal à la largeur de la bande.

7. Prothèse creuse implantable selon la revendication 1,
**caractérisée**
**en ce que** la pièce de la prothèse est formée en enroulant une bande orientée axialement d'une façon double, qui est en une matière ayant une proportion des pores de 45 à 94 %, une surface spécifique des pores de 0,1 à 0,6 µm²/µm³, une distance moyenne entre les pores de l'ordre de 5 à 45 µm et une valeur moyenne de la corde du volume de 5 à 30 µm.

8. Prothèse creuse implantable selon la revendication 1,
**caractérisée**
**en ce qu'**elle comporte un élément ( 3 ) de renfort qui est sous la forme de bagues qui sont fixées sur la surface extérieure de la prothèse.

9. Prothèse creuse implantable selon la revendication 1,
**caractérisée**
**en ce qu'**elle comporte un élément ( 3 ) de renfort sous forme de spirales qui sont fixées sur la surface extérieure de la prothèse.

10. Prothèse creuse implantable selon la revendication 8 ou 9,
**caractérisée**
**en ce que** les éléments ( 3 ) de renfort sont fixés sur l'enveloppe ( 4 ) extérieure supplémentaire qui est formée par l'enroulement de la bande en matière polymère et qui est appliquée de manière mobile le long de l'axe longitudinal de la prothèse.

11. Prothèse creuse implantable selon la revendication 8 ou 9,
**caractérisée**
**en ce qu'**elle comprend en outre une enveloppe ( 5 ) qui est formée en enroulant une bande en matière polymère fixée au dessus de l'élément ( 3 ) de renfort.

12. Prothèse creuse implantable selon la revendication 1,
**caractérisée**
**en ce que** la bande est en une matière polymère choisie dans le groupe qui comporte le polytétrafluoroéthylène et le copolymère tétrafluoroéthène avec de l'hexafluoropropylène, qui a une teneur de pas plus de 2 % en poids du copolymère tétrafluoroéthène avec de l'oxyde de perfluorovinylpropyle.

13. Prothèse creuse implantable selon la revendication 1,
**caractérisée**
**en ce que** la carcasse intérieure est en une matière polymère choisie dans le groupe qui comporte le polytétrafluoroéthylène et le copolymère tétrafluoroéthène avec de l'hexafluoropropylène ainsi que le tétrafluoroéthène avec de l'oxyde de perfluorovinylpropyle ayant une teneur du comonomère de pas plus de 2 % du poids.
